# EUROPEAN PATENT APPLICATION

(11) **EP 4 324 484 A1**
(43) Date of publication of application: **21.02.2024**
(21) Application number: 22191073.0
(22) Date of filing: 18.08.2022
(51) Int. Cl.: A61K 47/68, C07K 16/46

(54) **CATALYST AUGMENTED TARGET SPECIFIC POLYPEPTIDE**

(71) Applicant: Technische Universität Berlin, 10623 Berlin (DE)
(72) Inventor: Rappsilber, Juri, Berlin (DE); Belsom, Adam, Berlin (DE); Perez-Lopez, Ana, Berlin (DE)
(74) Representative: Fleuchaus & Gallo Partnerschaft mbB

(57) **Abstract**

The present invention relates to an improved targeted transition metal catalyst (TMC) suitable for human therapy.

## Description

The present invention relates to an improved targeted transition metal catalyst (TMC) for human therapy.

After the development of chemotherapeutic anticancer treatments based on cisplatin or its analogues in the late 1990's the knowledge around cytotoxicity, usefulness, effectiveness of transition metal based anticancer agents has increased dramatically and led - *iter alia* - to the rise of the field of bioorthogonal chemistry (van de L'Isle et al., 2021, Current Opinion in Chemical Biology, 61: 32-42). Remarkable developments and advances of transition metal catalysts (TMCs) as bioorthogonal tools have helped to explore, elicit and tune therapeutic approaches by medicinal chemistry.

Commonly the term "bioorthogonal" refers to artificial synthetic chemistry using highly chemospecific reactive partners conducted in a biological environment without causing adverse biological effects. In the context of the present application "bioorthogonal" is further used to describe compounds modified by synthetic chemistry and medicinal chemistry to make them applicable into a biological environment and suitable for human therapy.

TMCs (transition metal catalysts) are typical representatives for bioorthogonal chemistry.

TMCs comprising platinum, so called platinum complexes, are known to target DNA and are predisclosed as successful antitumor drugs. In contrast, it has been found that palladium complexes, even if from a chemical perspective significantly similar and thus promising, do so far not prove as valuable. Abu-Surrah et al., 2008, (Cancer therapy Vol. 6, pp 1-10) review the numerous palladium complexes with promising activity against cancer that have been synthesised. A key factor that might explain why palladium complexes are less useful, depends on their fast hydrolysis and ligand-exchange kinetic, which is about 10⁵ times faster than for platinum complexes. Such rapid dissociation in solution is leading to highly reactive, i.e. species too reactive to remain functionally able to reach the intended pharmacological target in a living subject.

It is thus a need in the prior art to provide further bioorthogonal catalysts that are protected from immediate reactivity when administered to a biological environment.

It is thus an objective of the present invention to provide alternative approaches to design TMC complexes suitable for therapeutic intervention in a subject in need thereof and/or suitable or favourable as therapeutic or diagnostic agents.

The object of the present application has been solved by the newly developed catalytic unit complexing a transition metal atom with a peptide motif according to claim 1 of the present invention. Dependent claims are directed to further embodiments or suitable applications of the claimed catalytic unit.

For this the inventors provided a catalytic unit comprising a bioorthogonal transition metal catalyst complexing an abiotic transition metal atom, which has the potential to catalyse chemical reactions and is advantageous in that the unit protects the catalyst from chemically reacting with biomolecules and thereby from being quickly deactivated by biomolecules.

Bioorthogonal chemistry represents a class of high-yielding chemical reactions that proceed rapidly and selectively in biological environments, largely without affecting biomolecules or interfering with biochemical processes.

According to the invention, the term "catalytic unit" defines primarily a chemical unit or i.e. a complex comprising an abiotic transition metal atom which is interacting with a peptide motif.

In chemistry, the terminology "transition metal" is - *iter alia* - used for defining different groups of metals in the periodic table. According to the IUPAC definition the term "transition metal" is primarily understood as any element in the d-block of the periodic table, which includes the atoms of the elements having between 0 and 10 d-electrons.

However, not all 40 elements of the d-block are relevant or suitable for the present invention and consequently the present invention, whenever using the term "transition metal" understands this as referring to the elements of the group 4d-5d, which due to their ability to form different oxidation states and their utilisation of 4d or 5d electrons in changing the activation energy in neighbouring chemical reaction often are referred to as catalysts.

Not all 20 elements of the group 4d and 5d are relevant or suitable for the present invention and consequently the present invention distinguishes between biotic and abiotic transition metal atoms and provides catalytic units with abiotic transition metal catalysts (TMC), only.

Often transition metal catalysed reactions or corresponding catalytic units comprise biotic transition metal atoms, such as Fe, Zn, Ni, Cu, Mg and Mn. However, the use of biotic transition metals present two important limitations. Achieving the desired catalytic effect in the context of a living system is challenging, as often the catalysis requires lethal temperatures and toxic metal concentrations. Secondly, the catalysts are not fully bioorthogonal, and metabolic enzymes can catalyse the same reactions at undesired destinations, contributing to elevated side effects. However, there has been limited success in the administration of TMC and thus the potential of their chemical reactions into a biological environment, as often reaction conditions needed by the TMC are incompatible with a biological environment, because they might require organic solvents and/or high temperatures or might lead to an immediate and thus unpredictable chemical reaction in the biological environment.

In contrast to this, the catalytic unit of the present invention employs abiotic transition metal atoms, which allow mediation of non-natural chemical reactions including deprotections or ligations at mild conditions (in isotonic solution, pH=7.4, 37 °C). The catalysis of reactions which do not occur in living systems, enables the production of active molecules in situ from harmless precursors, and it can be a powerful tool for labelling proteins, activating sensors, synthesising drugs, repairing tissues, or modulating biological functions.

Abiotic metals are metals not derived or a part of living organisms. In contrast, biotic metals such as Fe, Zn, Ni, Cu, Mg and Mn represent those metals that are part of biological systems.

Accordingly, the transition metal catalysts that are of interest for the present application are selected for the catalytic units, according to the present invention, from the abiotic transition metals of the group of 4d-5d transition metals. In particular the present invention refers to palladium and the different states of Pd such as Pd(ll) and/or Pd(0). According to the experiences of the inventors the metal oxidation states mentioned are suitable, since some oxidation states are unstable (i.e. not long-lasting). Others may be too reactive, for example oxidising too quickly in water, which would render them unsuitable for use in biological systems.

Due to the effort of the inventors, it became possible to develop a novel form of complexing the preferred abiotic TMC into a catalytic unit, which is at the same time stabilising and protecting the catalytic potential of the TMC and as such can be exploited, tested and optimised for biological environments, specialised medical uses and novel treatments.

For the catalytic unit of the invention the inventors used a peptide motif, which can be synthesised e.g. as cDNA, or any other suitable nucleic acid molecule and further can be multiplied, isolated and purified according to standard methods.

Said peptide motif is then incubated with the transition metal. While incubating the isolated peptide motif in an aqueous solution comprising the atoms of the TM of choice a stable complex is formed. These complexes are the catalytic units and can be isolated as catalytic units e.g. by the standard method of self-assembled metal complexation (Assembled metal complexation described below). Such catalytic units can be stored in suitable buffers or can be directly used, administered or tested.

The peptide motifs of the present invention are synthesised and provided as nucleic acid sequence defining the selected and specific amino acid peptide motif. The peptide motif selected for complexing the transition metal may vary but will be identified hereinafter as "complexing peptide motif".

Suitable complexing peptide motifs follow a general teaching, which can be best described by the following consensus sequence, which comprises at least 3-50 amino acids, which contain histidine, methionine, tryptophan, cysteine or tyrosine residues as binding sites (B), preferably two separated by 1-4 amino acids and following the pattern B(X)ₙB, n = 1-4.

According to one embodiment the present invention provides for complexing Pd into a catalytic unit according to the present invention a complexing peptide comprising the motif "HRGDH" (SEQ ID No: 1).

According to one embodiment, the complexing peptide motif has a binding affinity of at least Kd 10⁻⁵ M towards the transition metal ion. The inventors found that a binding affinity starting at a value of Kd10E-5 M, preferably at a value of Kd 10E-6 M or higher, is ideal for the catalytic unit, as with such binding stability the catalytic unit is much more stable than an unprotected or chemically protected transition metal catalyst. Such a catalytic unit can thus be used in a biological context without being toxic to the biological context, i.e. without immediately activating the catalytic potential and thus without immediately starting a catalytic reaction, which could then cause biological damage and would be considered toxic.

Accordingly, the catalytic unit protected by the complexing peptide, which has a Kd of at least 10E-8 M, is particularly stable in physiological fluid or solution and thus can travel or be administered to the location to be treated without starting a physiologically toxic catalytic reaction in the biological system or e.g. at the immediate entry site.

The catalytic unit of the present invention is thus protected, which is a substantial improvement, and allows the targeting of a catalytic unit and thus the catalytic reaction to a location (e.g. a tumour) to be treated, which may be far from the immediate entry site. Such protected catalysts will not be activated or deactivating at immediate contact with biological molecules and thus, before the catalytic unit can reach a suitable target site.

Additionally, according to one embodiment the peptide motif may be elongated by direct synthesis, fusion or ligation to additionally comprise a further peptide, polypeptide or protein sequence, which have a targeting functionality. Such peptide, polypeptide or protein selected for its targeting functionality will be identified as "targeting polypeptide" throughout the present invention.

Targeting and the necessity for improving targeting technologies is a long existing need in the various approaches of cancer treatment. With the development of tumour specific antibodies targeting a tumour with therapeutic approaches has been substantially improved. Over the years it was demonstrated that antibody based antitumor therapies have a conceptual advantage over standard chemotherapy, e.g. by increasing specificity, by offering various treatment options, namely different mechanisms of action to kill cancer cells such as including antibody dependent cellular cytotoxicity (ADCC) or phagocytosis (ADCP), complement-mediated cytotoxicity (CMC) and direct induction of apoptosis.

However, it is often seen that highly specific antibodies suitable to target specific tumours have a low efficiency, for example due to poor stability in the circulatory system or poor tissue penetrability. Even large amounts of mAbs administered to reach the effective concentration in the tumour will not compensate for the known stability issues, which significantly reduces treatment tolerability. This has been observed for treatments using HER2-antibodies, where large amounts of HER2-antibodies reached only in insignificant amounts, lung cells expressing low-levels of HER2. Consequently, the main limitation for their clinical use is presently the low efficiency of the tumour targeting antibodies.

Attempts to increase the efficiency of mAbs have included combination with chemotherapy (i.e. trastuzumab in combination with a taxane, typically docetaxel). However, trastuzumab plus chemotherapy is associated with additional adverse effects that are comparable to, or even worse than, chemotherapy alone. Another example is rituximab in combination with HDAC inhibitors. Again, more adverse events were reported than with just chemotherapy or the antibody alone.

Still another approach to combine antibodies and chemotherapy is fusing the cytotoxic drug to an antibody, resulting in an antibody-drug conjugate (ADC). ADCs are involved in more than 160 clinical trials, 10 of which have been commercially approved by the FDA. Presently reported such ADC therapies are still subject to limitations, including low uptake in the tumour (0.003-0.08%), low stability in plasma and low payload of drugs. Therefore, huge doses are required with subsequent inherent toxicity, while achieving high-doses is also a fundamental challenge that needs addressing.

Alternative ways to try to increase the drug dose inside tumours included increasing the loading of drug molecules per antibody, with drug loading increased to up to eight molecules compared to the standard four. The consequently highly potent ADC, Enhertu (AstraZeneca) consists of a HER2-antibody, trastuzumab, fused to deruxtecan by way of maleimide cysteine bioconjugation.

Still, another approach works with antibody-directed enzyme prodrug therapy (ADEPT) using antibodies fused to an enzyme catalyst, overcoming the limitation of drug loading for ADCs. High local concentrations of active drugs are achieved since an enzyme can generate thousands of drug molecules per molecule of antibody. Cowling et al, 2021, provides a comprehensive review regarding particularly the problems of palladium catalyst targeting options.

There are three major problems associated with ADEPT: i) the lack of bioorthogonality because the catalytic reactions are mediated by bacterial enzymes but can also be catalysed by endogenous human enzymes; ii) immunogenicity against the bacterial enzyme; iii) poor tumoral penetrability due to the large protein structure (>50KDa).

It is for the efforts of the inventors that an alternative approach to increase the efficiency and the tolerability of a catalyst augmented tumour treatment are now provided. By complexing the catalytic unit according to the invention with a suitable peptide motif, which then can be fused to various tumour targeting polypeptides or antibodies, the known and above-mentioned disadvantages can be overcome, and the therapeutic effects are substantially improved.

The present invention thus provides a solution for adding a catalytic capability to a delivery molecule while maintaining the catalytic activity, without poisonous effects in the patient, and maintaining the function of the delivery molecule or targeting molecule without impairing its binding capabilities.

As mentioned above the present invention provides a method of complexing an abiotic TMC with a protective peptide motif, which according to further embodiments can be elongated, fused, linked or joined by further molecular or genetic techniques to a targeting polypeptide, e.g. a tumour specific antibody or other targeting molecule.

Such targeting polypeptide may, according to some embodiments, be separated from the TM complexing peptide motif by a suitable linker. Known suitable linkers are typically G-rich sequences of 1-8 repetitive G-rich motifs. Alternatively, sequences defined as (GGG)n, (GGGS)n, or (GGGGS)n, can be used as linkers and/or spacers between the peptide motif complexing the TM and the peptide or polypeptide with targeting functionality.

Such targeting polypeptide has the capability to add the target specific functionality of a protein, e.g. a peptide, antibody, single domain antibody, nanobody or binding fragments thereof to the biorthogonal transition metal catalytic unit of the invention. For this, suitable target specific molecules are selected from the group of therapeutic antibodies, antigen-specific antibodies, tumour specific antibodies, antibody fragments, Fab, Fab2, F(ab')2, scFv, diabodies, single domain antibodies, target peptide and nanobodies.

Typically, targeting polypeptide according to the present invention are specific to known cell-specific surface structures, tumour antigens or targets, which can be selected from the group comprising, but not limited to HER2 (metastatic breast cancer), CD59 (B-cell chronic lymphocytic leukaemia), PD-L1 (urothelial carcinoma, metastatic non-small cell lung cancer, metastatic Merkel cell carcinoma), VEGF (metastatic colorectal cancer, non-squamous non-small-cell lung carcinoma, glioblastoma, metastatic renal cell carcinoma, cervical cancer), CD19 (precursor B-cell acute lymphoblastic leukaemia), CD30 (Hodgkin lymphoma, anaplastic large-cell lymphoma), EGFR (metastatic colorectal carcinoma, metastatic squamous non-small cell lung carcinoma), CD38 (multiple myeloma), GD2 (paediatric neuroblastoma), SLAMF7 (multiple myeloma), CD20 (B-cell non-Hodgkin's lymphoma, chronic lymphocytic leukaemia, follicular lymphoma, diffuse large B-cell lymphoma), CTLA4 (metastatic melanoma), PD-1 (metastatic squamous non-small cell lung carcinoma, metastatic melanoma), VEGFR2 (gastric cancer), CD22 (precursor B-cell acute lymphoblastic leukaemia) and CD33 (acute myeloid leukaemia).

Such targeting polypeptide or target specific molecule will maintain their target specificity in the status of being complexed to the catalytic unit. Simply binding assays will be employed to prove and ensure that the catalytic unit is truly protecting the catalytic potential of the TM to avoid a destruction of the targeting molecule and additionally, can prove the stability and maintenance of the binding specificity of the targeting molecule complexed with the TM.

In such a context of targeted catalytic treatments, biorthogonal approaches are considered to be potentially interesting to transform a bioorthogonal chemical into a bioactive material. One example would be to work with so-called prodrugs, which are precursors of active agents that are converted by a catalytic unit to the active agent following the administration to a patient.

Typically, prodrugs are based on active agents that have been protected with a cleavable protecting group or pro-moiety. By providing an active agent precursor that produces the active agent within the body, compounds can be produced that exhibit improved pharmacokinetic properties compared to the active drug, such as greater oral bioavailability or sustained release profiles. For safety reasons, it is desirable to provide prodrugs that do not exhibit biological activity themselves. The activity profile of the prodrug is then entirely dependent on the metabolic conversion of the prodrug to the active agent, providing a greater degree of predictability of biological activity *in vivo.*

Typical prodrugs are selected from the group of propargylated anticancer drugs of the active agent paclitaxel, doxorubicin, SN-38 (active metabolite of irinotecan), gemcitabine, 5-fluorouracil (5-FU), vorinostat, panobinostat, floxuridine, olaparib , pemetrexed, sunitinib, nintedanib, mitoxantrone, 4-hydroxytamoxifen, etoposide, duocarmycin and derivatives thereof.

Of particular interest in the context of the invention is ProPTX as a prodrug of paclitaxel, a potent microtubule inhibitor used as the first-line treatment drug in breast cancer, that presents a bioorthogonal gap up to 300-fold in HER2+ breast cancer SK-BR-3 cells.

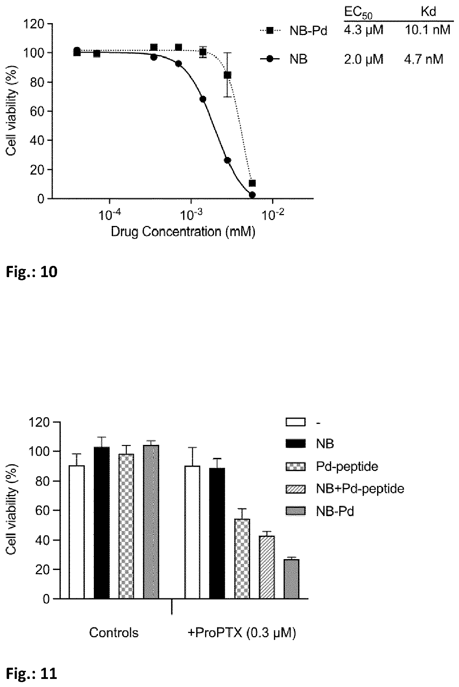

Particularly the synergistic effects demonstrated in Figure 11 show the potential of the herein described therapeutic approach.

Furthermore, the present invention provides the method to efficiently and reproducibly assemble the catalytic unit complexing the TM of choice. For this, in a first method step, the nucleic acid molecule, e.g. cDNA encoding the peptide motif is amplified.

In case a target specific catalytic unit is built, the nucleic acid encoding the peptide motif is elongated by the nucleic acid sequence of a target specific molecule and optionally the two motifs are joined by a suitable spacer or linker.

Then the amplified sequence is translated *in vitro* or *in vivo,* e.g. *E.coli,* into a peptide or polypeptide, which is isolated and optionally purified by well-known standard methods.

The incubation step then pairs the purified polypeptide and a suitable amount of metal atom of choice, preferably in the form of a pharmaceutically acceptable salt or other soluble salt combined in a suitable or pharmaceutically acceptable buffer. Suitable or preferred soluble salts of palladium are selected from, but not limited to, Pd(OAc)₂, Pd(Cod)Cl2, PdCl2, (CF3COO)2Pd or PdSO4. Using the peptide motifs with or without targeting molecule according to the indicated sequence above the TM complexes in a form of self-assembly with the peptide motif into the catalytic unit of the present invention and can then be isolated and/or separated from precursor molecules by ultra-centrifugal filter molecular weight cut-off.

This self-assembly process is highly advantageous in comparison to chemical bioconjugations is simpler, needs less protective chemical components and still leads to a structurally well-defined conjugate incorporating the metal of choice in the highly affinity peptide motif.

The present invention provides further a pharmaceutical composition comprising the catalytic unit as described above and a pharmaceutically acceptable carrier, diluent or excipient.

This pharmaceutical composition, the isolated catalytic unit itself and preferably the target-specific catalytic unit are provided for the use in the treatment of soluble, solid, benign, malignant and/or metastatic tumours selected from tumours with established target sites/antibody binding sites.

HER2+++ breast cancer metastasizes preferentially in bone, lung, liver and brain. The current treatment for metastatic breast cancer is the combination of trastuzumab and pertuzumab (anti-HER2 monoclonal antibodies) and a single-agent chemotherapeutic. Beyond trastuzumab, the incorporation of other anti-HER2 drugs in daily clinical practice, such as pertuzumab, trastuzumab-emtansine (T-DM1), and lapatinib, has achieved median overall survival of approximately 5 years, and a substantial proportion of patients (approximately 30%-40%) are alive at 8 years. However, it is still challenging to treat patients with HER2-positive brain metastasis (overall survival 1 year) where the administration of a prodrug of 5-FU was reported with acceptable blood brain barrier permeability would be used in combination with the target-specific catalytic unit for HER2, preferably a HER2-nanobody previously reported for crossing the blood brain barrier and currently in clinical trials phase I and II for radionuclide therapy (Puttemans et al., Cancers, 2020).

Furthermore, pharmaceutical composition, the isolated catalytic unit itself and preferably the target-specific catalytic unit are provided for the use in the treatment of disorders connected with the existence or dominance of specific cell types for which established target sites/antibody binding sites have been identified.

The target sites/antibody binding sites are overexpressed receptors in the most common cancers (i.e. HER2, VEGF, CD19, etc), but in addition there are targetable proteins/receptors in neurodegenerative diseases such as aggregated Aβ forms predominantly accumulated in Alzheimer disease or α-synuclein in Parkinson's disease. Currently the monoclonal antibodies (mAbs) Gantenerumab and Cinpanemab, against Aβ forms and α-synuctein respectively, are in clinical trials phase II and III. Additionally, several monoclonal antibodies to treat autoimmune diseases (i.e. rheumatoid arthritis, Crohn's disease, ulcerative colitis, etc) are available that specifically block proinflammatory cytokines. These include mAbs specific to tumour necrosis factor (TNF), interleukin (IL)-1β, IL-6, IL-17 and IL-12/IL-23.

As a preferred embodiment the pharmaceutical composition, the isolated catalytic unit itself and preferably the target-specific catalytic unit will be used in the treatment of cancer and will be co-administered with other cancer therapeutic agents, thermotherapy, plasmonic material, chemotherapeutic agents and/or prodrugs to a person in need thereof.

For example, attempts to increase the efficiency of mAbs have included combination with chemotherapy (i.e. trastuzumab in combination with docetaxel). Another approach to combine antibodies and chemotherapy is fusing the cytotoxic drug to an antibody, resulting in an antibody-drug conjugate (ADC) (i.e. trastuzumab fused to deruxtecan). Catalytic units itself or preferably the target-specific catalytic unit will be administered in combination with propargylated prodrugs of the active agent paclitaxel, doxorubicin, SN-38 (active metabolite of irinotecan), gemcitabine, 5-fluorouracil (5-FU), vorinostat, panobinostat, floxuridine, olaparib , pemetrexed, sunitinib, nintedanib, mitoxantrone, 4-hydroxytamoxifen, etoposide, duocarmycin and derivatives thereof.

### Short description of the figures

Figure 1 shows the structure of the catalytic unit complexing the catalyst and being fused to a spacer and a targeting polypeptide. (1) targeting ligand; (2) a spacer and optional sequence and (3) a binding sequence for the abiotic metal Pd.
Figure 2 shows the expression vector for the molecular generation and amplification of the peptide motif according to the invention, in form of a descriptive plasmid scheme of HER2-nanobody fused to the polypeptide (a spacer GGGGS (SEQ ID No: 2) and the binding metal site HRGDH) on the vector pETM11. The plasmid contains a TEV site sequence and 6xHis for purification (sequence of 13 amino acids ENLYFQSHHHHHH (SEQ ID No: 3)).
Figure 3 a) shows the process to identify peptides binding metals from *E.coli* lysates to the analysis by LCMS.

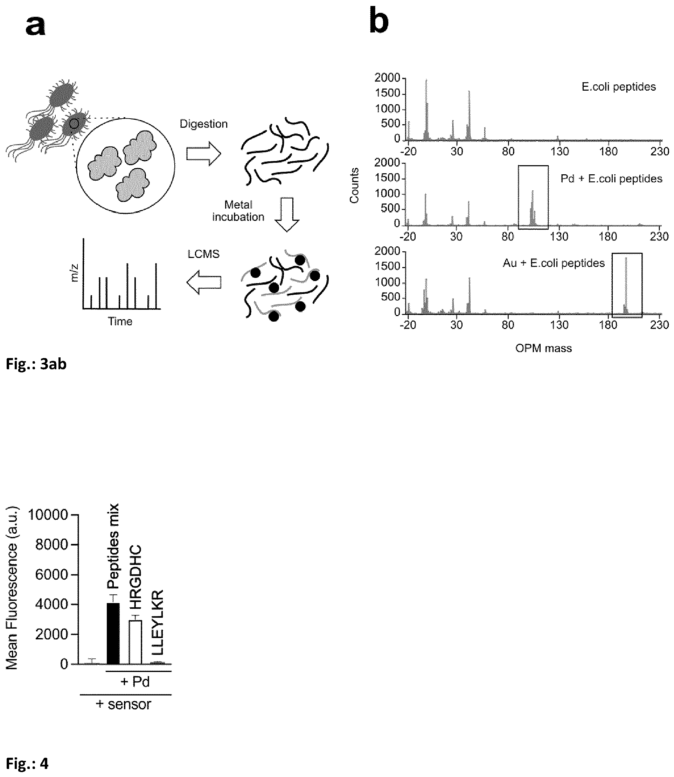

Figure 3 b) shows the open-modification search (OPM) of the peptide mass in *E.coli* lysates after incubation with Pd(ll) or Au(III). The squares highlight the cluster of peptides binding Pd or Au (approx. 1400 peptides). These metal binding peptides are stable, surviving LCMS conditions including the gas phase, which allows their analysis by electrospray ionisation high-resolution mass spectrometry.
Figure 4 shows the fluorescence signal for the catalytic conversion of the propargylated resorufin (ProRes, 100 µM) into the fluorescent resorufin by Pd-peptides (10 µM) in PBS after incubation at 37°C for 24 h. The mix of peptides from *E.coli* lysates and the peptide HRGDHC not only binds Pd, they show catalytic activity as well. However, the peptide LLEYLKR despite binding Pd does not show any catalytic activity.

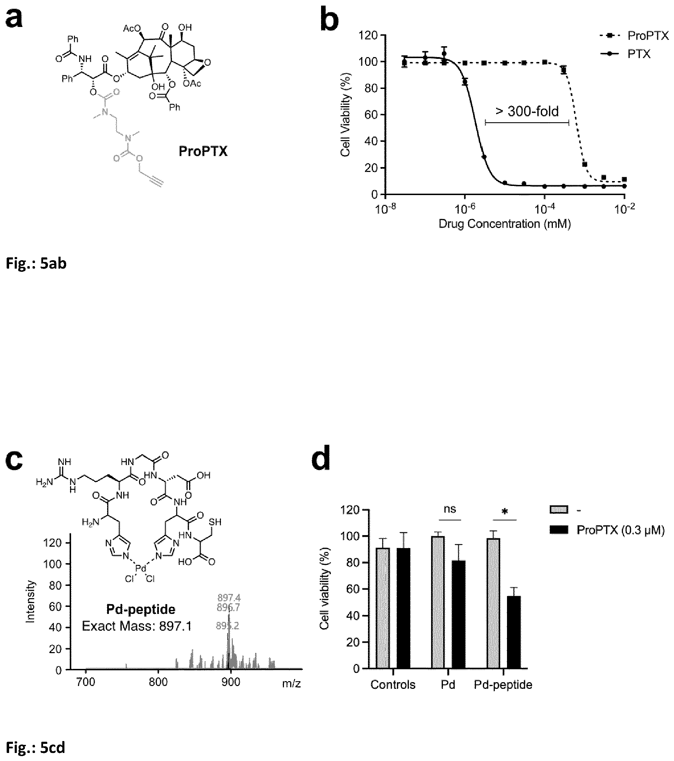

Figure 5 a) shows the structure of paclitaxel prodrug (ProPTX) with the aliphatic OH at C2' position masked by a propargyl-N,N'-dimethylethylenebicarbonate.
Figure 5 b) shows the semilog dose-response curves of paclitaxel versus paclitaxel prodrug for SK-BR-3 breast cancer cells.
   Paclitaxel potency was reduced more than 300-fold in the prodrug form (EC₅₀: PTX = 1.8 nM, ProPTX = 626 nM). This vast drop of anticancer activity highlights the essential role of the C2'-OH of PTX for binding its target.
Figure 5 c) shows the mass spectrum of the bis-His palladium-peptide complex (HRGDHC-PdCl,). The peptide HRGDHC was synthesised following established peptide synthesis procedures. The peptide HRGDHC was incubated with Pd(Cod)Cl₂, in water at 1:1 molar ratio. The HRGDHC-PdCI, complex (Pd-peptide) was purified by reverse phase SPE.
Figure 5 d) shows SK-BR-3 breast cancer cells incubated with the combination of bis-His palladium-peptide complex (Pd-peptide, 1.4 µM) and paclitaxel prodrug (ProPTX, 0.3 µM) converting the prodrug to clinically FDA-approved microtubule inhibitor paclitaxel (PTX, 0.3 µM). Conversely, Pd source (Pd(Cod)Cl₂, 1.4 µM) did show a poor catalytic efficiency. Error bars: LSD from n =2. Significance was determined by one-way analysis of variance (ANOVA): ^{ns}P > 0.05, ^{∗}P < 0.05.

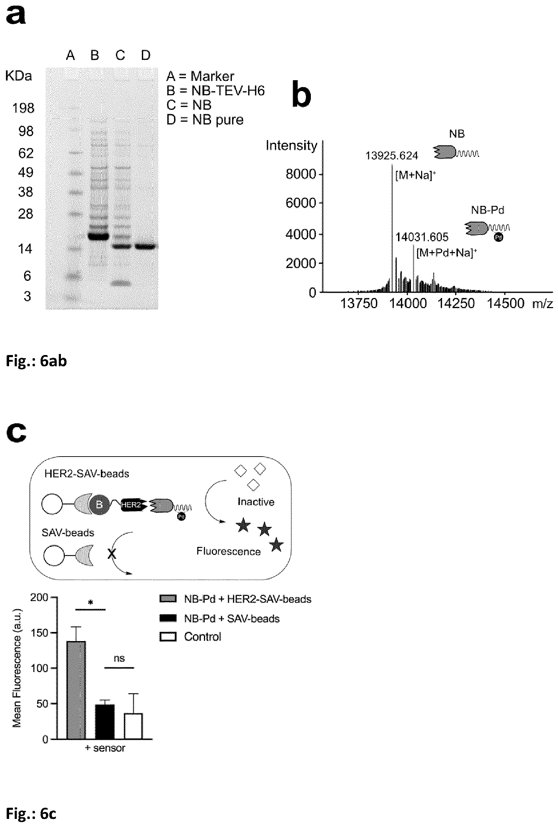

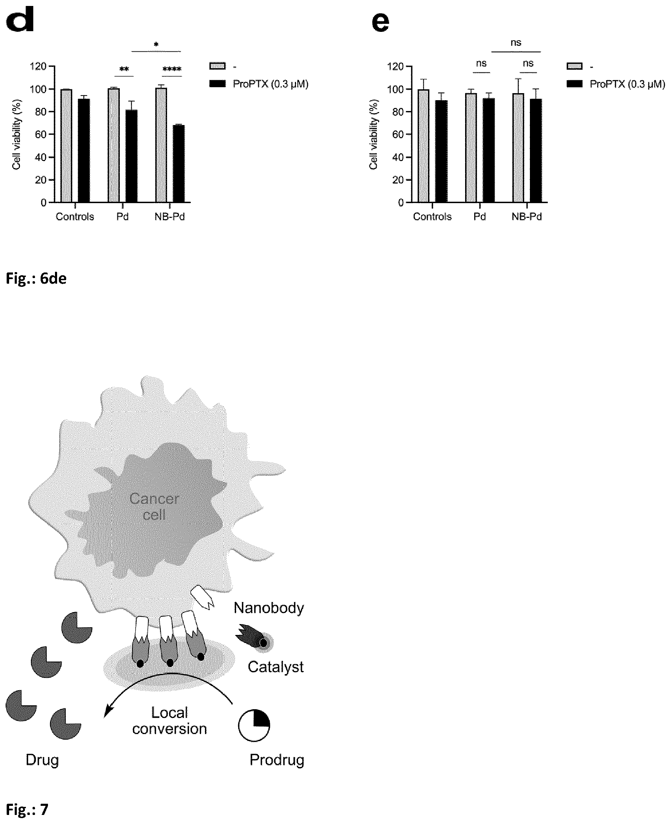

Figure 6 a) shows the SDS-PAGE of HER2-Nanobody-HRGDH fusion protein purification: A. Marker; B. Nanobody-HRGDH-TEVsite-His6 before TEV cleavage; C. Nanobody-HRGDH-TEVsite-His6 after TEV cleavage; D. TEV protease; E. Flow-through of Ni-NTA purification; F. Nanobody-HRGDH. Nanobody-HRGDH-TEVsite-H6 expressed in *E. coli* and purified by Ni-NTA chromatography. The fusioned protein was incubated with TEV protease to get the final constructor (NB-HRGDH).
Figure 6 b) is a mass spectrum of the NB-HRGDH-Pd (NB-Pd, 2.5 µg/mL) after incubation of NB-HRGDH (NB) with Pd(OAc)₂, at a molar ratio 1:1.
   The peaks corresponding to NB and NB-Pd were observed [M+Na]+ and [M+Pd+Na]+, respectively.
Figure 6 c) shows the catalytic deprotection of the propargylated dye (ProRes, 100 µM) results in the presence of NB-Pd + SAV-beads (20 nmol) and NB-Pd + HER2-SAV-beads (20 nmol) at physiological conditions (PBS, pH 7.4, 37°C). An increment of the fluorescence signal was observed by the resorufin release.
Figure 6 d) shows the catalytic activation of a prodrug (ProPTX, 0.3 µM) by NB-HRGDH-Pd (NB-Pd, 20 µg/mL, 1.4 µM) in HER2+ breast cancer cell line SK-BR-3. Cell viability was measured on day 5 using PrestoBlue reagent. Error bars: LSD from n =3. Significance was determined by one-way analysis of variance (ANOVA): ^{ns}P > 0.05, ^{∗}P < 0.05, ^{∗∗} P < 0.01, ^{∗∗∗∗} P < 0.0001.
Figure 6 e) shows the catalytic activation of a prodrug (ProPTX, 0.3 µM) by NB-HRGDH-Pd (NB-Pd, 20 µg/mL, 1.4 µM) in HER2- breast cancer cell line MCF-7. Cell viability was measured on day 5 using PrestoBlue reagent. Error bars: LSD from n =3. Significance was determined by one-way analysis of variance (ANOVA): ^{ns}P > 0.05, ^{∗}P < 0.05, ^{∗∗} P < 0.01, ^{∗∗∗∗}P < 0.0001.
Figure 7 shows the core principle of Catamab therapy.

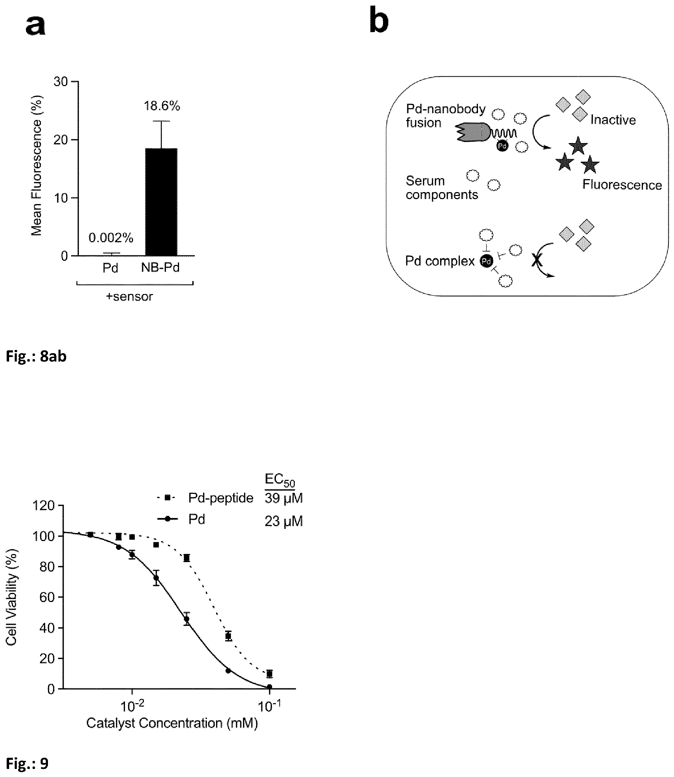

Figure 8 shows the fusion of Pd to the protein (nanobody) protecting the catalytic activity of Pd from deactivation by serum. (a) Catalytic deprotection of the propargylated dye (ProRes, 100 µM) occurs in the presence of either Pd(OAc)₂ (Pd, 1.4 µM), or in the presence of NB-HRGDH-Pd (NB-Pd, 1.4 µM). The catalysis was performed at physiological conditions (pH 7.4, 37 °C) in PBS or in serum (10% FBS in PBS). The fluorescence measurements were normalised against the value of fluorescence after catalysis in PBS (100%) and corrected by the fluorescence of the product, Resorufin, in PBS and serum, respectively. (b) Scheme representing the catalysis of ProRes by NB-Pd in the presence of serum and the catalytic deactivation of Pd(OAc)₂ by serum.
Figure 9 shows the peptide-supported Pd has increased biocompatibility compared to the free metal. Dose-response curves of Pd (Pd(OAc)₂) and Pd-peptide (HRGDHC-Pd) at different concentrations (100-1 µM) in HER2+ breast cancer cell line SK-BR-3. Cell viability was measured on day 5 using PrestoBlue reagent. Error bars: ±SD from n=3. Pd toxicity was reduced after fusion with the polypeptide (EC₅₀ 39 µM for Pd and 23 µM for Pd-peptide).
Figure 10 shows the dose-response curves of NB and NB-Pd at different concentrations (5.6-0.04 µM) in HER2+ breast cancer cell line SK-BR-3. Cell viability was measured on day 5 using PrestoBlue reagent. Error bars: ±SD from n=3. NB toxicity was slightly reduced after binding with the Pd (EC₅₀ 2 µM, Kd 4.7 nM for NB and EC₅₀4.3 µM Kd 10.1 nM for NB-Pd).
Figure 11 shows the localisation of the Pd-catalyst by the nanobody to cell surfaces enhances the efficacy of the drug in a synergistic manner. Catalytic uncaging of a paclitaxel prodrug (ProPTX, 0.3 µM) in the presence of either naked Nanobody (NB), Pd-peptide, NB/Pd-peptide or NB-Pd (1.4 µM), in HER2+ breast cancer cell line SK-BR-3. Cell viability was measured on day 5 using PrestoBlue reagent. Error bars: ±SD from n=3. The Pd-peptide in combination with ProPTX demonstrates the effect from catalysis alone. Application of NB with separate Pd-peptide in combination with ProPTX shows the summed effect of catalysis by the Pd-peptide and the apoptotic effect of the NB. The greatest reduction in cell viability however is caused by the synergistic effect of the NB-Pd in combination with ProPTX, which exceeds the simple sum of individual components.

### Detailed description of employed methods

Chemicals and solvents were purchased from Thermo Fisher Scientific, Sigma-Aldrich, VWR International Ltd, abcr Germany, Axon Medchem, ChemPUR. Prodrug of paclitaxel (ProPTX) and on/off resorufin sensor ProRes were synthesised following published procedures. The ¹H-NMR spectral data matched the values reported in the literature.^{1,2} Fmoc-amino acids (Fmoc-Cys(Trt)-OH, Fmoc-His(Trt)-OH, Fmoc-Gly-OH, Fmoc-Arg(Pbf)-OH, Fmoc-Asp(OtBu)-OH) were purchased from GL Biochem (Shanghai) Ltd. NMR spectra were recorded at ambient temperature on a 500 MHz Bruker Avance III spectrometer. Chemical shifts are reported in parts per million (ppm) relative to the solvent peak. *R*_{f} values were determined on Merck TLC Silica gel 60 F254 plates under a 254 nm UV source. Purifications were carried out by the Biotage Selekt flash column chromatography or C18 reverse phase chromatography. Compounds purity was >95% as measured by either TLC and NMR. MS was performed in an Advion expression compact mass spectrometer (CMS) (Ion source ESI). Protein MS analysis was done in a 12T Bruker SolariX Fourier Transform Ion Cyclotron Resonance Mass Spectrometry (FT-ICR MS). Stock solutions (100 mM) were prepared in DMSO.

*Bacterial culture media composition.* LB-medium (10 g tryptone, 5 g yeast extract, 10 g NaCl per litre) (AppliChem, A0954) was used to culture bacteria. LB-medium with agar (15 g agar per litre) (AppliChem, A0927) in plates was used to isolate individual (clonal) colonies of bacteria carrying a specific plasmid.

*Lysate buffer composition (pH 7.4, 4°C*). 50 mM HEPES, 10% Glycerol, 200 mM NaCl, 10 mM Imidazole, 10% Glycerol, 2 mL of 10x protease inhibitors (cOmplete protease inhibitors (EDTA-free) 1 tablet in 20 mL), 0.2 µL of benzonase (Millipore, E1014), 0.5 mg of lysozyme from chicken egg white (Sigma-Aldrich, L6876).

*Ni-NTA buffers composition (pH 7, 4°C).* 50 mM HEPES, 200 mM NaCl, 10% Glycerol, 10 mM Imidazole (Buffer A) or 400 mM Imidazole (Buffer B).

*Hi-Prep desalting buffer (pH 7, 4°C).* 50 mM HEPES, 200 mM NaCl, 10% Glycerol (Buffer C).

*Cell culture conditions.* SK-BR-3 and MCF-7 cells were cultured in RPMI-1640 and DMEM respectively, both supplemented with 10% of fetal bovine serum and L-glutamine (2 mM) and maintained in a tissue culture incubator at 37°C and 5% CO₂.

### Mass spectrometric analysis and identification of metal binding peptides

*E.coli K-12* cells were harvested, sonicated and the soluble proteins were precipitated with acetone. Peptides were separated on a NuPAGE 4-12% Bis-Tris gel using MES running buffer and bands were excised from the gel. Proteins were reduced with 20 mM DTT, alkylated using 55 mM IAA and digested using trypsin overnight at 37 °C, following standard protocols.³ Digested peptides were extracted from gel pieces, acidified to pH 2 with addition of TFA and desalted using self-made C18 Stagetips.⁴) Peptide mixtures (10 µM) were incubated with metallic additives (Pd(OAc)₂, HAuCl₄, RuCl₂(NH₃)₆, Pt(NH₃)₂Cl₂) (10 µM) in PBS (100 µL) for 1 h at 37 °C in a Thermomixer 1200 rpm. The mixture of metallopeptides were isolated following established Stagetip protocols and analysed using a Q Exactive HF mass spectrometer (Thermo Fisher Scientific, Bremen, Germany) coupled to an Ultimate 3000 RSLC nano system (Dionex, Thermo Fisher Scientific, Sunnyvale, USA), running 0.1% (v/v) formic acid (mobile phase A) and 80% (v/v) acetonitrile, 0.1% formic acid (mobile phase B). Peptides were loaded in 1.6% acetonitrile, 0.1% formic acid on an Easy-Spray column (C18, 50 cm, 75 µm ID, 2 µm particle size, 100 Å pore size) operated at 45 °C and running 300 nl/min with a gradient: 5 min linear increase from 2% B to 7.5% B; linear increase to 42.5% B in 80 min; linear increase to 50% B in 2.5 min; 2.5 min increase to 90% B and 5 min increase to 95%. The following settings were applied in the mass spectrometer: MS1 scan resolution 60,000, AGC (automatic gain control) target 3 × 10⁶, maximum injection time 100 ms, scan range from 400 to 1450 *m*/*z.* The ten most abundant precursor ions with z = 2-6 passing the peptide match filter ("preferred") were selected for HCD (higher-energy collisional dissociation) fragmentation employing a normalised collision energy of 28. Quadrupole isolation window was set to 1.4 *m*/*z.* Minimum AGC target was 1 × 10⁴, maximum injection time was 100 ms. Fragment ion scans were recorded with a resolution of 15,000, AGC target set to 1 × 10⁵, scanning with a fixed first mass of 100 *m*/*z.* Dynamic exclusion was enabled for 30 s and included isotopes. Raw files were processed into peak lists using msConvert (version 3.0.11729)⁵ including denoising (top 20 peaks in 100 *m*/*z* bins) and conversion to mgf-file format. Precursor masses were re-calibrated to account for mass shifts occurring during measurement. Resulting peak files were analysed using xiSEARCH 1.6.746⁶ with the following settings: MS1/MS2 error tolerances 3 and 5 rpm, respectively, allowing up to two missed trypsin cleavages, carbamidomethylation on cysteine as fixed and oxidation of methionine as variable modification, losses: -CH₃SOH/-H₂O/-NH₃, and crosslinker "OpenModification". The following "Custom Settings" were applied: "FRAGMENTTREE:FU", "OM_MIN_MASS:100" and "OM_MAX_MASS:500". Results were subjected to FDR (false discovery rate) processing, performing a 5% FDR calculation on peptide pair-level using a target-decoy based approach⁷ and lists of matched peptides, including peptides with additional mass corresponding to metal modification (Pd: 105.903475, Au: 196,966560) were written out as .csv format.

### Peptide synthesis

Wang resin (1 g, loading 1.2 mmol/g) was swollen in a minimum of DCM for 30 min. Fmoc-Cys(Trt)-OH (10 eq) or Fmoc-Arg(Pbf)-OH was dissolved in dry CH₂Cl₂ and a solution of DIC (5 eq) in dry CH₂Cl₂ was added. The mixture was stirred for 20 min at 0 °C, followed by removal of the CH₂Cl₂ in vacuo. The residue was dissolved in the minimum of DMF and the solution added to the swollen resin. DMAP (0.1 eq) in DMF was added to the resin mixture, which was agitated for 2 h. The resin was then filtered and washed with DMF (x3), CH₂Cl₂ (x3), MeOH (x3), Et₂O (x2) dried under vacuum for 30 min and the level of attachment estimated following standard quantitative Fmoc test.^{8,9} Fmoc removal was performed using 20% piperidine in DMF with two sequential treatments of 20 min. The resin was then filtered and washed with DMF (x3), CH₂Cl₂ (x3), MeOH (x3), Et₂O (x2) and dried under vacuum for 30 min. Resin was swollen in a minimum amount of CH₂Cl₂. N-Fmoc-amino acid (5 eq) and HBTU (4.9 eq) were dissolved in DMF (0.1 M). DIPEA (10 eq) was added and the resulting mixture was immediately added to the resin. The resin was agitated for 40 minutes to affect coupling. The resin was washed with DMF (x3), CH₂Cl₂ (x3), MeOH (x3), Et₂O (x2) and dried under vacuum for 30 min. The completion of each coupling was verified using the qualitative ninhydrin test. If needed, an extended cycle was repeated until completion of the reaction.

*Cleavage of peptide.* Resin was swollen to a minimum of CH₂Cl₂. TFA/TIS/H₂O (95/2.5/2.5) was added and the resin agitated for 2h. The TFA solution was removed, concentrated to ca. 1 mL and added to cold (4 °C) Et₂O in a centrifuge tube. The resulting precipitate was collected by centrifugation, washed with Et₂O (x4) and purified by the Biotage Selekt C18 reverse phase chromatography, flow 6 mL/min, gradient 5 to 100% B over 40 min. (A: H₂O 0.1% FA, B: CH₃CN). The collect fraction were lyophilised to afford the peptide HRGDHC as a white solid (383 mg). m/z C₂₇H₄₁N₁₃O₉S (ES+): 763.3 (M+K)⁺, 366.2 (M+K+H)²⁺. The peptide LLEYLKR was collected as a white solid (29 mg). m/z C₄₄H₇₅N₁₁O₁₁ (ES+): 934.5 (M+H)⁺, 467.7 (M+2H)²⁺, 312.2 (M+3H)³⁺ (100%).

*Synthesis of Pd-peptides.¹⁰* Peptide HRGDHC or LLEYLKR (100 µM) was incubated in the presence of Pd(Cod)Cl₂ or Pd(OAc)₂ (100 µM) in PBS (0.5 mL) at 37 °C, 1200 rpm in a Thermomixer for 2 h. The mixture was purified by C18 reverse phase cartridge to remove the excess of Pd(Cod)Cl₂ and analysed by MS. Pd-HRGDHC (Pd-peptide) m/z C₂₇H₄₁Cl₂N₁₃O₉PdS (ES+): 897.4 (M+H)⁺. Pd-LLEYLKR m/z C₄₄H₇₅N₁₁O₁₁Pd (ES+): 1040.4 (M)⁺.

### Production and purification of the HER2+ nanobody fused to the peptide HRGDH

The plasmid encoding the cDNA sequence of an HER2 nanobody fused to bis-His peptide with a His-tagged that includes a protease-site (3Cp) for subsequent tag removal (QVQLQESGGGSVQAGGSLKLTCAASGYIFNSCGMGWYRQSPGRERELVSRISGDGDTWHKESVKGRFTISQD NVKKTLYLQMNSLKPEDTAVYFCAVCYNLETYWGQGTQVTVSSGGGGSHRGDHENLYFOSHHHHHH (SEQ ID No: 4)) was obtained by gene synthesis through GeneArt service (Thermo Scientific). The sequence was cloned into *E. coli* expression vector pETM11 kanamycin A resistance and produced in 2 L *E.coli BL21 gold (DE3) pLysS.* Nanobody expression was induced overnight at 18 °C with 0.25 mM isopropyl-B-D-thiogalactoside (IPTG). The *E. coli* cells were then harvested and sonicated, and the supernatant was subjected to purification by Ni-NTA affinity chromatography followed by Hi-Prep desalting chromatography. The purified nanobody-peptide fusion construct was cleaved by TEV protease (Sigma Aldrich, T4455) ratio 1:100 protease:protein at 4 °C overnight. The resulting protein was purified by reverse Ni-NTA affinity chromatography followed by Hi-Prep desalting chromatography. The purity was analysed by SEC (size-exclusion chromatography) using Buffer C and SDS-PAGE. The VHH solution (NB) was determined by measuring the protein concentration by UV absorption (ε 27,180 M⁻¹ cm⁻¹) at 280 nm and stored in aliquots (50 µg/125 µL) at -20 °C. FT-ICR MS for NB protein (M+Na)⁺: calcd 13925.303, found 13925.624.

### Assembled metal complexation

HER2+ nanobody fused to HRGDH peptide (50 µg, 3.5 nmol) was incubated with Pd(OAc)₂ (785 µg, 3.5 nmol) in 200 µL of Buffer C at 37 °C for 10 min in a gentle shaking at 200 rpm in the Thermomixer. The solution was diluted in PBS (200 µL) and purified using Amicon ultra-centrifugal filter unit (0.5 mL, MWCO 3 kDa) in a Thermo Scientific Heraeus Pico 17 benchtop centrifuge (14000 rpm, 10 min), and washed with PBS (2 × 0.5 mL) to elute unbounded Pd(OAc)₂. The concentrated protein solution NB-Pd (100 µL) was collected in an Eppendorf tube by centrifugation (1 min, 14000 rpm). FT-ICR MS for NB-Pd protein (M+Na)⁺: calcd 14031.206, found 14031.605.

### Fluorogenic studies

*Catalytic screening of metallopeptides.* Metallopeptides, either mixed or as individual Pd-peptides (Pd-HRGDHC or Pd-LLEYLKR) (10 µM) were incubated with Pd-labile off/on sensor O-propargyl-resorufin (ProRes)² (100 µM) in PBS (500 µL). The mixtures were shaken at 700 rpm and 37 °C in a Thermomixer and reactions were monitored after 24 h using a FLUOstar Omega multilabel reader (Ex/Em: 540/590nm).

*In solution.* 2.8 µg of NB-Pd were added to a 200 µL solution of a Pd-labile off/on sensor O-propargyl-resorufin (ProRes)² (100 µM) in PBS or in 10% FBS-PBS to obtain a final concentration of 1.4 µM of NB-Pd. The mixtures were shaken at 700 rpm and 37 °C in a Thermomixer, and reactions were monitored after 24h using a FLUOstar Omega multilabel reader (Ex/Em: 540/590nm). Sensor ProRes alone (100 µM), or in the presence of Pd(OAc)₂ (1.4 µM), were used as controls and fluorescent dye Resorufin (100 µM) was used as a reference control. Reactions were carried out in triplicate. The fluorescence measurements were normalised against the value of fluorescence after catalysis in PBS (100%) and corrected by the fluorescence of the product, Resorufin, in PBS and serum, respectively.

*On HER2-biotinylated streptavidin beads.* 6 µg of Her2/ERBB2 Protein, Human, Recombinant (ECD), Biotinylated (SinoBiological, 10004-HCCH-B) were dissolved in 100 µL PBS. HER2 protein solution was added to previously washed Streptavidin Sepharose High Performance affinity resin (50 µL, 0.4 nmol/µL) (Cytiva). The resin suspension was shaken at 700 rpm and 37 °C in a Thermomixer for 1h. Biotin (200 µM) in PBS (100 µL) was added and incubated for 30 min at 37 °C, 700 rpm in the Thermomixer to block free Streptavidin. Resin was washed twice with PBS and NB-Pd (1.6 nmol, 100 µL) was added. After incubation at 700 rpm and 37 °C in a Thermomixer for 1h, resin was washed twice with PBS and ProRes (100 µM) in PBS (100 µL) was added. Resin was incubated at 1200 rpm and 37 °C in a Thermomixer for 24 h. Fluorescence was measured in a FLUOstar Omega multilabel reader (Ex/Em: 540/590nm). Sensor ProRes alone (100 µM), or in the presence of Streptavidin Sepharose resin pre-incubated with NB-Pd, were used as negative controls and fluorescent dye Resorufin (100 µM) was used as a reference control. Reactions were done in triplicates.

### Cell viability study

The antiproliferative activities of Pd(OAc)₂ and Pd-peptide, or NB and NB-Pd were compared by performing dose-response studies against SK-BR-3 cells. Cells were seeded in a 96-well plate format (at 2,000 cells per well) and incubated for 24 h before treatment. Each well was then replaced with fresh media containing compounds (Pd(OAc)₂ and Pd-peptide (1-100 µM) or NB-Pd and NB (5.6-0.04 µM)). Untreated cells were incubated with DMSO (0.1% v/v). After 5d of incubation, cell viability was determined as described above. All conditions were normalised to the untreated cells (100%) and curves fitted using GraphPad Prism using a sigmoidal variable slope curve. Experiments were performed in triplicates. Kd of NB-Pd (1.01×10E-8 M) was estimated from the EC₅₀ values and the published Kd of HER2-NB (4.74 ×10E-9 M).¹¹

### Prodrug-into-drug conversion studies

*Intracellular conversion.* SK-BR-3 and MCF-7 cells were seeded in a 96-well plate format (at 2,000 cells per well) and incubated for 24h before treatment. Each well was then replaced with a solution of NB-Pd (20 µg/mL, 1.4 µM) in culture media and incubated for 1h. Cells were then washed with PBS and fresh media containing either DMSO (0.1% v/v) or ProPTX (0.3 µM) was added. The cells treated with PTX and ProPTX at 0.3 µM were used as positive and negative controls, respectively. After 5d, PrestoBlue cell viability reagent (10% v/v) was added to each well and the plate was incubated for 90 min. Fluorescence emission was detected using a FLUOstar Omega multilabel reader (Ex/Em: 540/590nm). Experiments were performed in triplicates.

*Extracellular conversion.* SK-BR-3 cells were seeded in a 96-well plate format (at 2,000 cells per well) and incubated for 24 h before treatment. Each well was then replaced with a solution of NB-Pd (20 µg/mL, 1.4 µM) or Pd-peptide (1.4 µM) in culture media and treated either DMSO (0.1% v/v) or ProPTX (0.3 µM). The cells treated with PTX and ProPTX at 0.3 µM were used as positive and negative controls, respectively. After 5d, PrestoBlue cell viability reagent (10% v/v) was added to each well and the plate was incubated for 90 min. Fluorescence emission was detected using a FLUOstar Omega multilabel reader (Ex/Em: 540/590nm). Experiments were performed in triplicates.

### Examples

The following examples illustrate viable ways of carrying out the described method as intended, without the intent of limiting the invention to said examples.

### Example 1: Generation of recombinant cDNA expression vector with chosen peptide motif, a spacer and a target specific ligand.

A recombinant fusion of a selected peptide sequence (GGGS-HRGDH) to anti-HER2 nanobody in *E. coli* was performed. The peptide-nanobody cDNA sequence was obtained by gene synthesis through the GeneArt service. The sequence was then cloned into *E. coli* expression vectors to produce His-tagged fusion protein that included a protease-site (3Cp) for subsequent tag removal. The peptide-nanobody fusion construct was purified using nickel affinity chromatography and the purity confirmed by size exclusion chromatography (SEC). The fusion protein was characterised by FT-ICR MS.

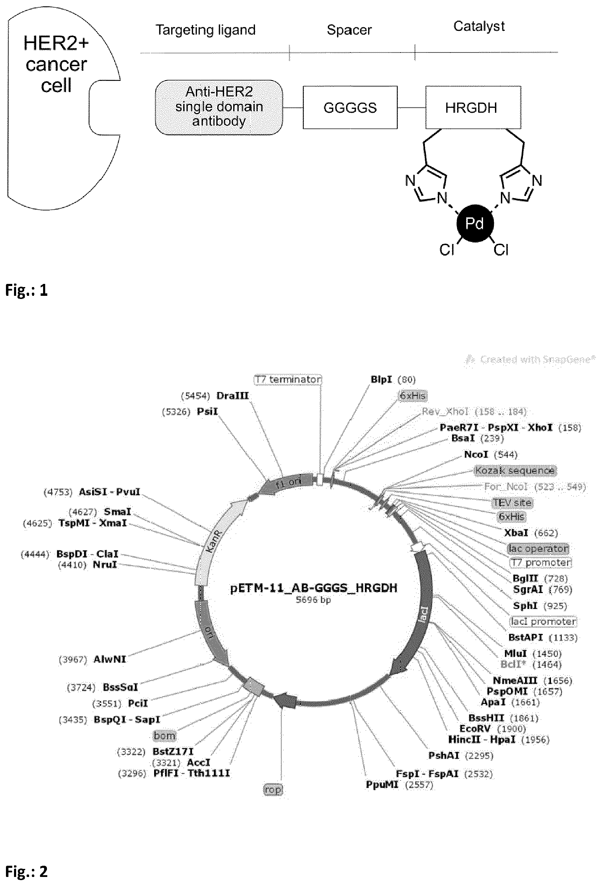

The general principle and a suitable vector are illustrated in Fig. 1 & Fig. 2. Characterisation are shown in Fig. 6ab.

### Example 2: Method of generation the catalytic unit

From cell lysates (*E.coli, HeLa cells),* proteins were extracted, digested and incubated with metallic additives (i.e. Pd(OAc)₂, Pd(OAc)₂(PPh₃)₂, PdCl₂(Cod), HAuCl₄, AuCl₃) in water or isotonic buffers for 1 h at 37 °C in a Thermomixer. The mixture of metallopeptides were isolated by StageTips and analysed by LCMS, following standard proteomic protocols. In short, peptide mixtures were separated by C18 chromatography on a nano-LC system, sprayed into a high-resolution mass spectrometer and subjected to electrospray ionisation. Peptides were analysed using a data dependent acquisition method, applying a "high-high approach", whereby both peptide precursors and subsequent peptide fragments, were analysed in the Orbitrap at high-resolution. All peptide sequences found binding to a given metal species were compared with/out metal and also comparing fragmentation spectra of metal-complexed and respective free peptides, as the metal species may influence the fragmentation behaviour of peptides around the complexation points, to delineate plausible sequence elements that facilitate metal complexation for future rational design.

The mixture of peptides bound metals (or metallopeptides) were incubated with a Pd-labile off/on sensor O-propargyl-resorufin (ProRes) in PBS or in 10% FBS-PBS at 37 °C in a Thermomixer, and reactions are monitored after 24 h using a multilabel reader (Ex/Em: 540/590 nm). The increment on the fluorescence signal validated the catalytic activity of the metallopeptides.

Results are to be found in Fig. 3 & Fig. 4.

### Example 3: Serum inhibition of Pd complex and protective effect of the peptide motif binding (OR nanobody binding)

Pd(OAc)₂ and Pd-peptide (Pd-HRGDHC) were incubated with the previously described sensor (ProRes) in PBS or in 10% FBS-PBS at 37 °C in a Thermomixer, and reactions were monitored after 24 h using a multilabel reader. Fluorescence increment was observed after catalysis. The catalytic activity of the metal in serum remained at least 18% of its catalytic activity in PBS when it was bound to the polypeptide while the free-metal lost its catalytic properties by interaction with proteins presence in the serum. Results to be found in Fig. 8.

### Example 4: Biocompatibility comparison

SK-BR-3 cells were incubated with Pd(OAc)₂ and Pd-peptide (Pd-HRGDHC) at different concentrations (1-100 µM) for 5 days. Presto blue reagent was added to quantitatively measure viability by fluorescence detection and EC₅₀ values were calculated by plotting dose-response curves. Pd bound to the peptide showed higher EC₅₀ value than the free-metal (39 µM and 23 µM, respectively), proving the protecting role of the peptide to avoid metal-protein interactions. Results are to be found in Fig. 9.

### Example 5: In vitro / in vivo data proofing functionality & catalytic activity of the catalytic unit to activate anticancer prodrug

SK-BR-3 cells were seeded and incubated with a solution of Pd(OAc)₂ or Pd-peptide (Pd-HRGDHC) in combination with either DMSO or ProPTX (0.3 µM). After 5 days, Presto blue cell viability reagent was added and fluorescence emission was detected using a multilabel plate reader. The combination treatment of Pd-peptide and ProPTX displayed >40% cell death confirming the catalytic conversion into the active form of paclitaxel. Non-significant catalysis was observed by free-Pd in combination with ProPTX proving that free metal becomes inactive in the presence of serum. Results are to be found in Fig. 5.

### Example 6: In vitro / in vivo data proofing functionality & catalytic activity of the target-specific molecule to activate anticancer prodrug in different cell lines

Streptavidin beads (SAV) were treated with the biotinylated HER2 protein (HER2-SAV-beads). Then, HER2-SAV-beads were incubated with the NB-Pd for 1 h at 37 °C in a Thermomixer. After washing with PBS, the sensor (ProRes) was added and the reaction was monitored after 24 h using a multilabel fluorescence reader. Increased fluorescence showed not only the catalytic conversion of the sensor but also the capability of the NB-Pd to simultaneously bind HER2 receptor.

SK-BR-3 and MCF-7 cells (HER2 positive and negative respectively) were seeded and incubated with a solution of NB-Pd for 1 h. Cells were then washed with PBS and fresh media containing either DMSO or ProPTX (0.3 µM) was added. After 5 days, Presto blue cell viability reagent was added and fluorescence emission was detected. The combination treatment of NB-Pd and ProPTX displayed >30% cell death in HER2 positive cells SK-BR-3 while no effect was observed in the HER2 negative cells MCF-7. Results are to be found in Fig. 6.

### Example 7: Synergistic effects and improvements

SK-BR-3 cells were seeded and incubated with a solution of (i) NB or (ii) Pd-peptide or (iii) NB and Pd-peptide or (iv) NB-Pd in culture media and treated with either DMSO or ProPTX (0.3 µM). After 5d, Presto blue cell viability reagent was added to each well and fluorescence emission was detected using a multilabel fluorescence reader. The combination treatment of Pd-peptide and ProPTX showed the effect of catalysis alone (>40% cell death).

NB with separate Pd-peptide in combination with ProPTX displayed the synergistic effect of catalysis by the Pd-peptide and the anticancer effect of the NB (>60% cell death). The combination of NB-Pd and ProPTX displayed >70% cell death caused by the synergistic effect of both NB-Pd and ProPTX, which exceeded the simple sum of individual components. Results are to be found in Fig. 11.

### References

1. Cowling et al., 2021, Bioorg. Med. Chem. 44, 116298
2. van de L'Isle et al., 2021, Current Opinion in Chemical Biology, 61: 32-42
3. Abu-Surrah et al., 2008, Cancer therapy Vol. 6, pp 1-10
4. Puttemans et al., Cancers, 2020.

## Claims

1. A catalytic unit comprising a bioorthogonal transition metal catalyst, wherein the transition metal catalyst is an abiotic transition metal atom which is in complex to a peptide motif, wherein the peptide motif protects the catalyst from being deactivated by binding to biomolecules or from chemically reacting with biomolecules, thereby providing a motive for biocompatible fusion of the transition metal catalyst to a targeting molecule.

2. The catalytic unit according to claim 1 wherein the transition metal catalyst is selected from Pd(ll) and/or Pd(0).

3. The catalytic unit according to claim 1 or 2 wherein the peptide motif has a binding capacity of at least Kd 10⁻⁶ M towards the metal ion.

4. The catalytic unit according to any of the claims 1 to 3 wherein the peptide motif is selected from the group of consensus sequence, which comprises at least 3-50 amino acids containing histidine, methionine, tryptophan, cysteine or tyrosine residues as binding sites (B), wherein two such binding sites are separated by 1-4 amino acids following the pattern B(X)ₙB, n = 1-4.

5. The catalytic unit according to any of the claims 1 to 4, wherein the peptide motif is encoded in a nucleic acid sequence selected from the group consisting of cDNA and/or synthetic nucleic acid sequences.

6. The catalytic unit according to any of the claims 1 to 5, wherein the catalyst is Pd and the peptide motif has the binding site defined by an amino acid sequence HRGDH.

7. The catalytic unit according to any of the claims 1 to 6, wherein the peptide is directly or via a linker linked or fused to a target specific molecule.

8. The catalytic unit according to claim 7, wherein the spacer is a G-rich spacer or selected from the group of spacers with the amino acid sequence [GGGGS], [GGGS] or [GGGGG]n.

9. The catalytic unit according to any of the claims 1 to 8, wherein the target-specific molecule is peptide or polypeptide selected from the group of therapeutic antibodies, antigen-specific antibodies, tumour specific antibodies, antibody fragments, Fab, Fab2, F(ab')2, scFv, diabodies, single domain antibodies, target peptide and nanobodies.

10. The catalytic unite according to any of the claims 1 to 9, wherein the target-specific molecule is specifically targeting cell-specific surface structures or tumour antigen, and/or antigens are selected from the group comprising HER2 (metastatic breast cancer), CD59 (B-cell chronic lymphocytic leukemia), PD-L1 (urothelial carcinoma, metastatic non-small cell lung cancer, metastatic Merkel cell carcinoma), VEGF (metastatic colorectal cancer, non-squamous non-small-cell lung carcinoma, glioblastoma, metastatic renal cell carcinoma, cervical cancer), CD19 (precursor B-cell acute lymphoblastic leukemia), CD30 (Hodgkin lymphoma, anaplastic large-cell lymphoma), EGFR (metastatic colorectal carcinoma, metastatic squamous non-small cell lung carcinoma), CD38 (multiple myeloma), GD2 (pediatric neuroblastoma), SLAMF7 (multiple myeloma), CD20 (B-cell non-Hodgkin's lymphoma, chronic lymphocytic leukemia, follicular lymphoma, diffuse large B-cell lymphoma), CTLA4 (metastatic melanoma), PD-1 (metastatic squamous non-small cell lung carcinoma, metastatic melanoma), VEGFR2 (gastric cancer), CD22 (precursor B-cell acute lymphoblastic leukemia), CD33 (acute myeloid leukemia).

11. Method of generating the catalytic unit according to any of the claims 1 to 10 comprising the steps of
- amplifying the cDNA of the peptide motif, optionally together with a suitable spacer and the sequence of a target specific molecule;
- purifying the polypeptide;
- exposing the purified polypeptide to a suitable amount of metal atoms in the form of a pharmaceutically-acceptable salt or soluble salt from Pd;
- incubating the purified polypeptide with the suitable metal salt in a pharmaceutically-acceptable buffer; and
- isolating the catalytic unit now in complex with the metal atom after co-incubation.

12. A pharmaceutical composition comprising the catalytic unit according to any of the claims 1 to 10, and a pharmaceutically acceptable carrier, diluent or excipient.

13. The catalytic unit according to any of the claims 1 to 10 for use in the treatment of soluble, solid, benign, malignant and/or metastatic tumours selected from tumours with established target sites/antibody binding sites.

14. The catalytic unit according any of the claims 1 to 10 for use in the treatment of disorders connected with the existence or dominance of specific cell types for which established target sites/antibody binding sites have been identified.

15. The catalytic unit for use in cancer therapy according to claim 13 and 14 co-administered with a cancer therapeutic agent, thermotherapy, plasmonic material and/or prodrug to a person in need thereof.
